# EUROPÄISCHE PATENTSCHRIFT

(11) **EP 2 490 611 B1**
(45) Veröffentlichungstag und Bekanntmachung des Hinweises auf die Patenterteilung: **21.06.2017**
(21) Anmeldenummer: 10776609.9
(22) Anmeldetag: 20.10.2010
(51) Int. Cl.: A61B 17/70, A61B 17/88, A61B 17/72

(54) **IMPLANTAT UND WERKZEUG FÜR DIE KYPHOPLASTIE**
IMPLANT AND TOOL FOR KYPHOPLASTY
IMPLANT ET OUTIL POUR LA CYPHOPLASTIE

(30) Priorität: 20.10.2009 DE 102009050142
(43) Veröffentlichungstag der Anmeldung: 29.08.2012
(73) Patentinhaber: Kraus, Kilian, 97440 Werneck (DE)
(72) Erfinder: Kraus, Kilian, 97440 Werneck (DE)
(74) Vertreter: Meissner Bolte Partnerschaft mbB
(86) Internationale Anmeldenummer: PCT/EP2010/065790
(87) Internationale Veröffentlichungsnummer: WO 2011/048140

(56) Entgegenhaltungen:
- WO-A1-2008/137192
- WO-A2-2007/078692
- US-A1- 2007 043 373

## Beschreibung

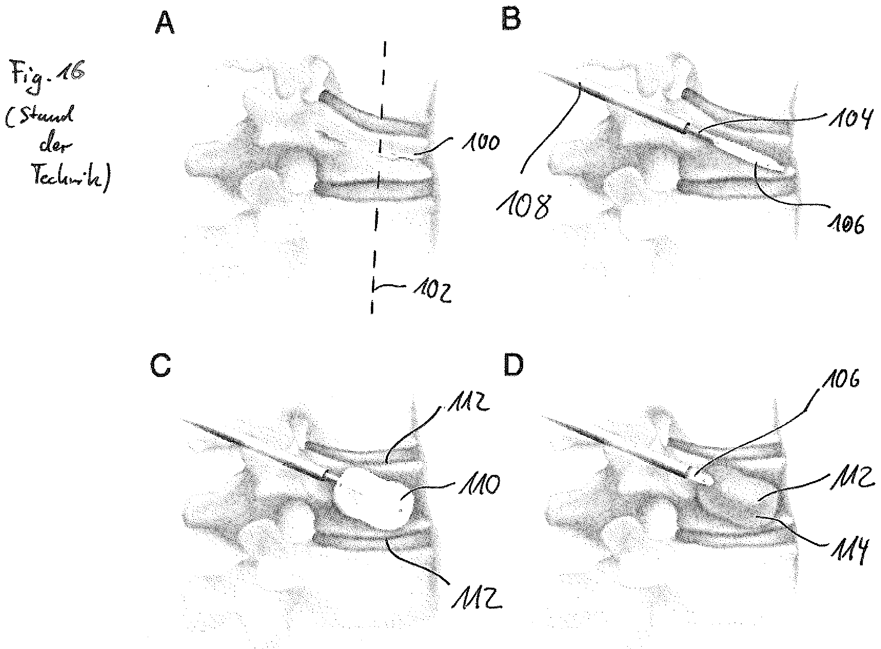

Implantat und Werkzeug für die Kyphoplastie Die Erfindung betrifft ein Implantat und ein Werkzeug für die Kyphoplastie. Fig. 16 illustriert ein als Kyphoplastie aus dem Stand der Technik bezeichnetes Verfahren. In Bild A ist ein Wirbelkörper 100 einer menschlichen Wirbelsäule gezeigt, der in Axialrichtung der Wirbelsäule, angedeutet durch die Linie 102, kollabiert ist. Durch ein Kyphoplastieverfahren soll dieser in seine ursprüngliche Form aufgerichtet und in dieser stabilisiert werden.
Dazu wird der Wirbelkörper 100 mit zumindest einer Bohrung 104 versehen, welche sich in das kollabierte Schwammgewebe (Spongiosa) hinein erstreckt. In die Bohrung 104 wird gemäß Bild B ein aufblasbarer Körper in Form eines Ballons 106 mit Hilfe eines Handhabungswerkzeuges 108 eingebracht. Der Ballon ist entleert und befindet sich damit in einem Ausgangszustand. Gemäß Bild C wird der Ballon mit Druckflüssigkeit 110 gefüllt, wobei er expandiert, das Spongiosa-Gewebe verdrängt und den defekten Wirbel 100 wieder so aufrichtet, dass seine zu jeweils benachbarten Wirbeln weisenden Flächen 112 etwa wie im ursprünglichen Zustand ausgerichtet sind. Der Ballon 106 wird also in einen Expansionszustand gebracht, bei dem dieser eine Volumenvergrößerung erfährt. Der Ballon 106 wird durch Ablassen der Druckflüssigkeit 110 wieder in seine ursprüngliche Form - wie in Bild B dargestellt - gebracht, also zurück in den Ausgangszustand transformiert, und gemäß Bild D aus dem Wirbelkörper 100 entfernt. Anschließend wird der durch das Aufblasen des Ballons 106 entstandene Hohlraum 112 im Wirbelkörper 100 mit einer aushärtenden Vergussmasse 114 gefüllt.

Bei einem weiteren aus dem Stand der Technik bekannten Verfahren wird in eine sich in den Wirbelkörper hinein erstreckende Bohrung ein auf mechanische Weise expandierbarer Körper als Implantat eingebracht. Das Implantat bzw. der Expansionskörper ist im Ausgangszustand ein zylindrisches Teil, das eine Mittellängsachse aufweist. Die Mantelfläche des Zylinders ist durch netzartig miteinander verbundene Stege gebildet. Nach Platzierung im Wirbelkörper wird mit Hilfe eines zum Implantat passenden Handhabungswerkzeugs das Implantat in einen Expansionszustand gebracht. Hierzu wird die axiale Länge des Expansionskörpers verkürzt, d.h. Deckel und Boden des Zylinders werden aufeinander zu bewegt. Die netzartig verbundenen Stege wölben sich hierdurch in Radialrichtung expandierend nach außen. Hierdurch wird ein Effekt wie beim o.g., mit Druckflüssigkeit befüllbaren Ballon erzielt. Der Expansionskörper, also das Implantat verbleibt allerdings im Gegensatz zum Ballon nach seiner Transformation in den Expansionszustand, also der Volumenvergrößerung im Wirbelkörper. Der das Implantat aufnehmende Hohlraum wird ebenfalls mit einer aushärtenden Vergussmasse gefüllt.

Nachteilig bei diesem Verfahren ist z.B., dass es nahezu unmöglich ist, den einmal verformten, also expandierten Expansionskörper aus dem Wirbelkörper wieder zu entfernen. Ein Entfernen ist aber beispielsweise dann erforderlich, wenn es sich zeigt, dass trotz maximaler Volumenvergrößerung des Expansionskörpers der Wirbel nicht zufriedenstellend aufgerichtet werden kann. Der bekannte Expansionskörper kann zwar durch das Handhabungswerkzeug wieder in die Länge gezogen werden, wobei sich sein Umfang verringert; aufgrund des verwendeten metallischen Materials (z.B. Titan) lässt sich aber eine vollständige Rückverformung auf den ursprünglichen Durchmesser des Expansionskörpers, nicht mehr erreichen. Grund hierfür ist die plastische Verformung des Bodens und Deckels, also die Endstücke des Implantats, verbindenden Netzes bei der Expansion. Durch ein erneutes Auseinanderbewegen der Endstücke - ausgehend vom Expansionszustand - ist zwar ein Teil der Verformung wieder rückgängig zu machen, jedoch nicht vollständig. Es verbleibt ein radial erweitertes Implantat.
Die Bohrung im Wirbelkörper zum Einbringen des Implantats sollte jedoch so knapp bemessen sein, wie möglich, um den Patienten zu schonen bzw. die Stabilität des Wirbelkörpers nicht weiter zu schwächen. Der Durchmesser des Implantats im Ausgangszustand entspricht daher dem Durchmesser der Zugangsöffnung im Wirbelkörper. Durch diese ist das einmal expandierte und wieder kontrahierte Implantat dann nicht mehr entfernbar.

Ein Implantat mit den Merkmalen des Oberbegriffs des Anspruchs 1 ist aus der WO 2008/137192 A2 bekannt. Aufgabe der Erfindung ist es, ein verbessertes Implantat, Verfahren und Werkzeug für die Kyphoplastie anzugeben. Hinsichtlich des Implantats wird die Aufgabe gelöst durch ein Implantat für die Kyphoplastie gemäß Patentanspruch 1. Das Implantat weist zwei Endstücke auf. Diese sind bezüglich einer Mittellängsachse des Implantats koaxial und mit einem Axialabstand zueinander angeordnet. Das Implantat weist außerdem einen Käfig auf, welcher die Endstücke verbindet und dabei die Mittellängsachse konzentrisch umgibt. Der Käfig enthält eine Mehrzahl von sich länglich erstreckenden Stegen, wobei jeder Steg die beiden Endstücke verbindet. Die Stege sind nur mit den Endstücken, also nicht untereinander verbunden. Mit anderen Worten sind die jeweiligen beiden Enden eines Steges jeweils mit einem der Endstücke verbunden. Das Implantat kann sich in einem Ausgangszustand befinden. In diesem sind die jeweiligen beiden Enden eines jeden Steges in Umfangsrichtung bezüglich der Mittellängsachse um einen gleichen Ausgangswinkel gegeneinander versetzt. Das Implantat ist auch, ausgehend vom Ausgangszustand, in einen Expansionszustand bringbar. Dies geschieht durch eine gegensinnige Rotation der beiden Endstücke um die Mittellängsachse. Die Rotation erfolgt in demjenigen Drehsinn, dass sich die Umfangspositionen der jeweiligen beiden Enden eines Steges aufeinander zu bewegen.

Die Mittellängsachse definiert eine Einführrichtung des Implantates in einen Patienten. D.h., dieses wird durch axiale Verschiebung entlang der Mittellängsachse in den Patienten eingebracht. Das in Einführrichtung weisende Endstück ist daher als vorderes Endstück bezeichnet und z.B. als Kuppe, Halbkugel, Kalotte oder ähnliche Form ausgebildet, um leicht in den Patienten bzw. eine für das Implantat vorgesehene Bohrung einführbar zu sein. Im oben genannten nicht expandierten Ausgangszustand sind deshalb die Stege so angeordnet, dass das gesamt Implantat einen maximalen Durchmesser nicht überschreitet. Das Implantat hat z.B. etwa die Umrissform eines geraden Kreiszylinders. In der Regel verlaufen daher im Ausgangszustand die Stege entlang einer Mantelfläche des Kreiszylinders, wobei die beiden Endstücke Deckel und Boden des Kreiszylinders darstellen. Ein jeweiliger Steg verläuft dann entlang einer Schraubenlinie, die Stege sind also schraubenförmig gewunden. Denkbar ist jedoch auch, dass das Implantat im Ausgangszustand etwa eine Form eines Tonnenkörpers oder Rotationsellipsoids aufweist. Die Stege verlaufen dann entlang dessen Mantelfläche. In der Regel besitzen auch alle Stege identische Form und sind in der Regel gleichmäßig über den Umfang der Mittellängsachse verteilt.

Bei der Expansion, also der Transformation vom Ausgangs- in den Expansionszustand bewegen sich die anfänglich in Umfangsrichtung versetzten Enden eines jeweiligen Steges zur gleichen Umfangsposition hin aufeinander zu. Die Stege bewegen sich radial nach außen. Der Außendurchmesser des Implantats nimmt zu. Im maximalen Expansionszustand liegen dann die Enden der Stege an der gleichen Umfangsposition, d.h. der Stege verläuft etwa in Axialrichtung. Das Implantat nimmt dann z.B. etwa die Umrissform eines Tonnenkörpers an. Die Stege werden hierbei entweder auf der gesamten Länge in sich verformt, hauptsächlich jedoch an ihren Verbindungsstellen zu den Endstücken. Die Verformung erfolgt hierbei in der Regel plastisch, so dass nach Beendigung der Bewegung das Implantat mechanisch stabil im Expansionszustand verharrt und nicht zurückfedert. Je nachdem, wie weit die Endstücke zueinander rotiert werden, sind verschiedene Expansionszustände und damit verschiedene Außendurchmesser realisierbar.

In der Regel ist das Implantat derart gestaltet, dass die Transformation vom Ausgangs- zum Expansionszustand keinesfalls zu einer Verkürzung der axialen Länge des Implantats, sondern allenfalls zu einer geringen Verlängerung führt. Da die Transformation durch Rotation der Endstücke erfolgt, ist auch eine Rücktransformation vom Expansions- in den Ausgangszustand durch entsprechendes Verdrehen der Endstücke in Gegenrichtung möglich. Das erfindungsgemäße Implantat bietet hierbei den entscheidenden Vorteil, dass es vollständig in den Ausgangszustand, also die Ausgangsform zurückdrehbar ist, d.h. wieder den maximalen anfänglichen Durchmesser aufweist. Insbesondere ist dies möglich, wenn im Ausgangszustand zwischen zwei benachbarten Stegen - in Umfangsrichtung gesehen - ein Restspalt vorhanden ist. Die entsprechenden Spalte verlaufen dann beispielsweise ebenfalls schraubenlinienförmig entlang der Mantelfläche des o.g. geraden Kreiszylinders. Wegen des Restspaltes ist beim Zurückdrehen in den Ausgangszustand ein geringfügiges Überdrehen über den Ausgangszustand hinaus möglich, welches die elastische Verformungsgrenze des Implantatmaterials überschreitet, so dass der Ausgangszustand tatsächlich erreicht wird.

Gemäß einer vorteilhaften Ausführungsform sind bei dem Implantat im Ausgangszustand die Enden jedes Steges in Umfangsrichtung bezüglich der Mittellängsachse um einen Ausgangswinkel von 180° versetzt. Mit anderen Worten umrunden die Stege von ihrem einen zum anderen Ende hin den halben Zylinderumfang des Implantates. Die Windungshöhe einer entsprechenden Schraubenlinie entspricht dann dem doppelten Abstand der beiden Endstücke. Bei einem derartigen ursprünglichen Versatz ist ein größtmöglicher Aufspreizwinkel des Implantates, d.h. eine größtmögliche radiale Erweiterung der Stege zwischen Ausgangs- und Expansionszustand erreichbar.

In einer weiteren bevorzugten Ausführungsform sind beim Implantat die Enden eines Steges in einer eine Verformung begünstigenden Weise gegenüber dem restlichen Teil des Steges geschwächt. Ziel bei der Transformation ist es, dass sich die Stege selbst möglichst nicht verformen. Beim Implantat sollten sich daher möglichst nur die Bereiche, in denen die Stege in die Endstücke übergehen, bewegen. Dies kann durch z.B. durch eine gelenkige Verbindung der Stege mit den Endstücken erreicht werden. Die alternative genannte Schwächung ist jedoch in der Regel vorzuziehen. Mit anderen Worten ist das Verbindungsstück der Stege mit den Endstücken bewusst geschwächt, um dort eine leichte und gezielte Materialverformung der Stege bei der gegenseitigen Rotation der Endstücke zu erreichen. Die Schwächung kann auch z.B. durch eine Einkerbung der Stege erfolgen. Alternativ kann eine Materialaufweichung der Stegenden erfolgen, indem dort z.B. das Material gegenüber dem restlichen Steg nicht oder weniger gehärtet wird. So kann eine gezielte Verformung des Implantates bzw. Stege an einer bestimmten Stelle erreicht werden. Außerdem kann so erreicht werden, dass sich der restliche Teil des Steges nahezu nicht verformt.

Die Schwächung erfolgt vorzugsweise dadurch, dass die Stegenden im Querschnitt gegenüber dem Querschnitt des restlichen Steges verjüngt sind.

In einer bevorzugten Variante dieser Ausführungsform sind die Stege durch eine an nur einer Seite des Steges vorhandene Ausnehmung verjüngt. Eine "Seite" bedeutet in diesem Zusammenhang einen jeweils in Umfangsrichtung weisenden Randbereich eines Steges. Besonders vorteilhaft ist es, wenn sich die Ausnehmung an derjenigen Seite des Steges befindet, welche in diejenige Drehrichtung des Stegendes weist, welche zu einer Transformation vom Ausgangs- in den Expansionszustand führt. Mit anderen Worten ist die Ausnehmung also an der in die expandierende Drehrichtung weisenden Seite des Steges eingebracht. Eine Ausnehmung bedeutet z.B. bei einem im Querschnitt rechteckigen Steg, dass die jeweiligen drei restlichen Stegseiten bündig verlaufen und lediglich die die Ausnehmung aufweisende Stegseite an deren Stelle in den Steg hinein zurückspringt. Die Anbringung der Ausnehmung an der eben genannten Seite des Steges führt dazu, dass sich die Stegkanten bei einem im Querschnitt eckigen Steg in besonders günstiger Weise radial nach außen aufstellen.

Bei einer weiteren bevorzugten Ausführungsform weist in einem axialen Mittelbereich des Implantates bzw. Steges die Flächennormale einer nach außen weisenden Fläche jedes Steges lediglich eine Radial- und gegebenenfalls eine Axialkomponente auf. Dies gilt sowohl für den Expansions- als auch für den Ausgangszustand. Mit anderen Worten unterliegen die Stege im Mittelbereich, d.h. in der axialen Mitte des Implantates, welche in der Regel auch die Stelle der größten Radiusaufweitung darstellt, keinerlei Torsionsbewegung. Insbesondere bei Stegen mit eckigem Querschnitt wird so verhindert, dass sich beim Aufweiten Kanten des Steges nach außen drehen und z.B. am Patienten schaben. Mit anderen Worten bilden die Außenflächen der Stege also im besagten Mittelbereich Tangentenflächen an die Zylindermantel- bzw. Tonnenkörperform des Implantats.

Bei einer weiteren bevorzugten Ausführungsform weist ein erstes Endstück des Implantates, vorzugsweise das in Einführrichtung gesehen vordere, ein in Umfangsrichtung wirksames erstes Eingriffsmittel für ein Werkzeug auf. Das gegenüberliegende zweite Endstück weist eine Durchtrittsöffnung für das Werkzeug und zusätzlich ein in Umfangsrichtung wirksames zweites Eingriffsmittel für das Werkzeug auf. Das Werkzeug kann dann durch die Durchtrittsöffnung und durch das zweite Endstück im Inneren des Implantates zum ersten Endstück hin gelangen, um dort mit dem ersten Eingriffsmittel in Eingriff zu kommen. Gleichzeitig gelangt das Werkzeug auch mit dem zweiten Eingriffsmittel am zweiten Endstück in Eingriff. Mit Hilfe des Werkzeuges kann dann eine jeweilige Drehbewegung bzw. ein Drehmoment auf die Endstücke ausgeübt werden, um diese zwischen Ausgangs- und Expansionszustand gegensinnig zu rotieren. In einer Variante dieser Ausführungsform ist das erste Eingriffsmittel eine Ausnehmung im ersten Endstück und das zweite Eingriffsmittel ein Formschlusselement am Außenumfang des zweiten Endstückes. Mit anderen Worten umgibt dann das zweite Eingriffsmittel die Durchtrittsöffnung.
In einer weiteren bevorzugten Ausführungsform ist das Implantat einstückig ausgeführt. Dies ermöglicht beispielsweise die Herstellung des Implantates aus einem stabförmigen Vollmaterial durch Drehen, Bohren und Laserschneiden. Ein nichterfindungsgemäßes Verfahren für die Kyphoplastie wird ebenfalls beschrieben. Das Verfahren dient zur Betätigung eines erfindungsgemäßen Implantates. Gemäß dem Verfahren werden - ausgehend vom Ausgangszustand - zum Expandieren des Implantates die Endstücke derart gegensinnig um die Längsachse rotiert, dass sich die Umfangspositionen der Enden eines jeden Steges aufeinander zu bewegen. Die Endstücke werden dabei so lange bzw. weit rotiert, bis eine gewünschte radiale Expansion der Stege, also ein Expansionszustand mit gewünschter radialer Erweiterung, erfolgt ist. Zum Kontrahieren des Implantats werden die Endstücke derart gegensinnig um die Mittellängsachse rotiert, dass sich die Umfangspositionen der Enden eines jeden Steges voneinander weg bewegen. Dieses erfolgt ebenfalls, bis ein gewünschter Expansionszustand mit reduziertem Durchmesser oder der Ausgangszustand erreicht ist. Das Expandieren ist hierbei der Vorgang der Transformation vom Ausgangs- zum Expansionszustand hin, das Kontrahieren beschreibt die entsprechend gegenläufige Transformation. Weiterhin werden die Endstücke gegensinnig derart rotiert, dass der axiale Mittelbereich des Implantats an einer festen, z.B. raumfesten Umfangsposition verbleibt. Mit anderen Worten erfolgt eine gegensinnig symmetrische Bewegung der Endstücke, so dass der Mittelbereich mehr oder weniger in Umfangsrichtung ruht und alleine eine Radialbewegung ausführt. Die beiden Endstücke werden also bezüglich eines raumfesten Bezugsystems um den jeweils gleichen Winkel gegensinnig rotiert. Insbesondere wenn das Verfahren im Inneren eines Patienten durchgeführt wird, findet am Mittelbereich keine Umfangsbewegung der Stege statt, was besonders patientenschonend ist. Es treten z.B. keinerlei Abschabeffekte oder ähnliches an der Stelle der größten radialen Erweiterung der Implantates, nämlich im Mittelbereich auf. Hinsichtlich des Werkzeuges wird die Aufgabe der Erfindung gelöst durch ein Werkzeug für die Kyphoplastie gemäß Patentanspruch 10, das zur Bedienung eines erfindungsgemäßen Implantats dient. Das Werkzeug weist einen Handgriff auf und einen stabförmigen, eine Mittellängsachse aufweisenden Grundkörper. Am einen Ende des Grundkörpers bzw. an einem Längsende ist der Handgriff angeordnet, in der Regel am anderen Ende eine Aufnahme für das Implantat. Mit anderen Worten ist also das Implantat am vom Handgriff entfernten Ende des Grundkörpers aufbringbar. Ein Bediener, z.B. ein Chirurg, kann somit das Werkzeug am Handgriff halten, um das vordere Ende des Grundkörpers mit dem Implantat in das Innere des Patienten einzuführen. Das Implantat ist in bevorzugter Weise derart am Grundkörper aufnehmbar, dass die Mittellängsachsen von Grundkörper und Implantat zusammen fallen. Das Werkzeug weist außerdem zwei Drehmittel auf, wobei jedes der Drehmittel an je einem Eingriffsmittel des Implantates kraftschlüssig angreift. Die Drehmittel sind bezüglich der Mittellängsachse des Grundkörpers gegenläufig in beiden Richtungen rotierbar. Durch Betätigung der Drehmittel wird also bei aufgesetztem Implantat Drehmoment auf die Eingriffsmittel ausgeübt, um letztendlich die oben beschriebene Rotation der Endstücke um die Mittellängsachse, und damit die o.g. Expansion oder Kontraktion zu bewirken.

In einer bevorzugten Ausführungsform sind die Drehmittel bezüglich des Handgriffes gegensinnig rotierbar und bezüglich eines gleichen Winkelversatzes zwangsgeführt. Mit anderen Worten findet also bei festgehaltenem Handgriff eine derartige Bewegung der beiden Eingriffsmittel statt, welche gegensinnig und synchron bezüglich des Winkelversatzes verläuft. Hierdurch ist insbesondere die oben genannte Ausführungsform der Implantatbedienung möglich, bei welcher der Mittelbereich ruht und nur jeweils die Endstücke gegensinnig um den gleichen Winkelbetrag rotieren. Dies hat zur Folge, dass dann auch der Mittelbereich bezüglich des Handgriffes ruht.

In einer bevorzugten Ausführungsform weist das Werkzeug mindestens ein Betätigungselement auf, welches eines oder bevorzugt beide Drehmittel antreibt. In bevorzugter Weise ist ein derartiges Betätigungselement in der Nähe des Handgriffes oder direkt am Handgriff angeordnet.

In einer Variante dieser Ausführungsform ist das Betätigungselement ein Drehknopf, welcher um eine Drehachse drehbar ist, die quer zur Mittellängsachse des Werkzeuges verläuft. Auch diese Ausführungsform begünstigt das Ruhighalten des Handgriffs in einer bestimmten Umfangsposition, und die oben beschriebene Transformation des Implantates bei ruhendem Mittelbereich.

Für eine weitere Beschreibung der Erfindung wird auf die Ausführungsbeispiele der Zeichnungen verwiesen. Es zeigen, jeweils in einer schematischen Prinzipskizze:
- Fig.1: ein erfindungsgemäßes Implantat im Ausgangszustand in perspektivischer Schrägansicht,
- Fig.2: das Implantat aus Fig.1 im Expansionszustand in Seitenansicht,
- Fig.3: das Implantat aus Fig.1 beim Aufsetzen auf ein Werkzeug,
- Fig.4: das Implantat aus Fig.1 in Frontalansicht,
- Fig.5: ein Implantat gemäß Fig. 1 mit alternativen Eingriffsmitteln,
- Fig.6: das Werkzeug aus Fig.3 in Gesamtansicht
- Fig.7: das Werkzeug aus Fig.3 ohne Implantat,
- Fig.8: ein alternatives Werkzeug im Detailschnitt,
- Fig.9: das Werkzeug aus Fig.8 mit alternativem Implantat,
- Fig.10: das Werkzeug aus Fig.8 ohne Handgriff,
- Fig.11: das Werkzeug aus Fig.8 im Detail mit Implantat,
- Fig.12: das alternative Implantat aus Fig. 8 in Schrägansicht,
- Fig.13: das Implantat aus Fig.8 im Querschnitt,
- Fig.14: das Implantat aus Fig.8 im Längsschnitt,
- Fig.15: ein alternatives Implantat mit Gelenken und Rundstegen,
- Fig.16: ein Kyphoplastieverfahren gemäß Stand der Technik.

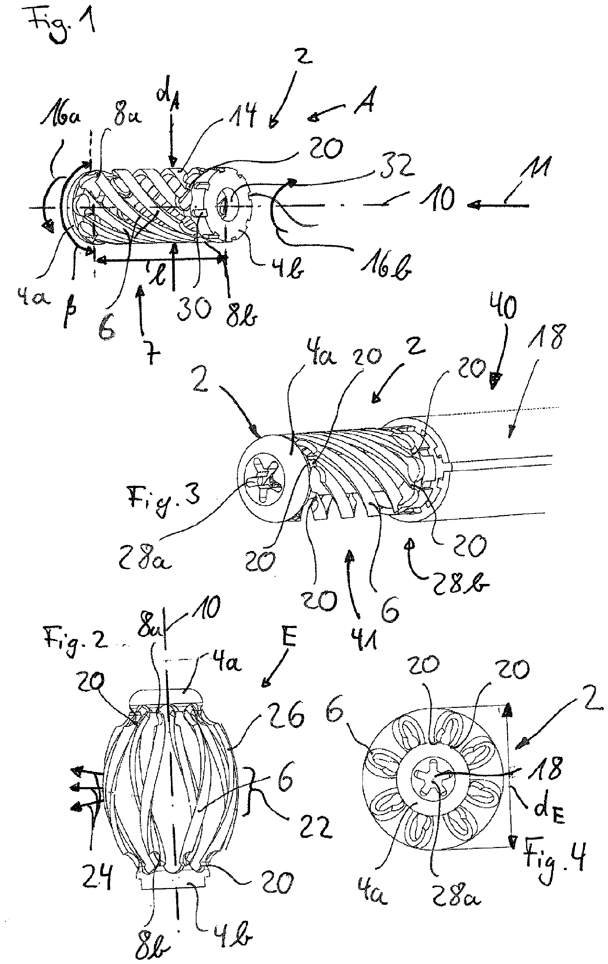

Fig. 1 zeigt ein erfindungsgemäßes Implantat 2 in einem Ausgangszustand A. Das Implantat hat im Wesentlichen die Form eines geraden Kreiszylinders. Boden und Deckel des Kreiszylinders werden durch ein erstes Endstück 4a und ein zweites Endstück 4b gebildet. Die Mantelfläche des Zylinders wird durch eine Mehrzahl von Stegen 6 gebildet, die zusammen einen Käfig 7 bilden. Diese winden sich in Form von Schraubenlinien auf dem Zylindermantel vom Endstück 4a zum Endstück 4b. Die Enden 8a,b jedes Steges sind mit den Endstücken 4a,b verbunden. Das Implantat besitzt eine Mittellängsachse 10. Mit anderen Worten sind also die Endstücke 4a,b über die schraubenförmig gewundene Stege 6 miteinander verbunden. Im nichtexpandierten Ausgangszustand A verlaufen die Stege an jeder Stelle derart parallel, dass ihre jeweiligen Tangenten bei der gleichen Axialhöhe parallel verlaufen. Jeder Steg 6 geht also - in Gedanken - bei entsprechender Rotation um die Mittellängsachse 10 in einen anderen Steg 6 über.
Im Ausgangszustand A besitzt das Implantat eine radiale Ausdehnung, die einen maximalen Durchmesser d_{A} nicht überschreitet. Das Implantat soll in Richtung des Pfeils 11 entlang der Mittellängsachse 10 in einen Patienten einführbar sein. Daher ist das erste Endstück 4a ein vorderes Endstück und in etwa als Kugelkalotte ausgeführt.
Im Beispiel sind die Stege 6 gleichmäßig über den Umfang des Implantats 2 verteilt und besitzen jeweils gleiche Abstände zueinander. Zwischen je zwei Stegen 6 ist also ein Restspalt 12 vorhanden, der ebenfalls die Gestalt einer Schraubenlinie hat. Der Abstand zwischen den Stegen 6, also die Breite des Restspaltes 12 in Umfangsrichtung ist kleiner als die entsprechende Breite der Stege 6. Die Stege 6 sind also so angeordnet, dass ihre Außenseiten 14 Teil einer gemeinsamen Zylinderhüllfläche sind.

Fig. 2 zeigt das Implantat aus Fig.1 in einem Expansionszustand E. Die Volumenvergrößerung des Implantats 2 gegenüber dem Ausgangszustand A erfolgt im Gegensatz zum Stand der Technik nicht durch eine Längenverkürzung des Implantats 2, also die Verringerung der axialen Länge l zwischen den Endstücken 4a,b. Die Transformation in den Expansionszustand E erfolgt dadurch, dass die beiden Endstücke 4a,b so gegeneinander verdreht werden, dass die Enden 8a,b der Stege 6 sich bezüglich der Umfangsrichtung der Mittellängsachse 10 aufeinander zubewegen. Die Rotation der Endstücke 4a,b erfolgt also bei der Expansion in Richtung der Pfeile 16a,b. Im Expansionszustand erreicht der Durchmesser d_{E} des Implantats ein Maximum.

Die entsprechende Drehung bzw. das nötige Drehmoment wird durch ein in Fig. 3 gezeigtes Werkzeug 18 erzeugt, auf das das Implantat 2 aufgesetzt ist. Fig. 2 zeigt allerdings eine Situation, in der das Implantat 2 noch nicht vollständig aufgesetzt ist. Das Werkzeug 18 verursacht also die Drehung der Endstücke 4a,b gegeneinander um die Mittellängsachse 10. Die Endstücke 4a,b werden dabei auf gleichbleibender Distanz der Länge 1 gehalten. Die Stege 6 wölben sich dabei radial nach außen und bewirken die gewünschte Volumenvergrößerung. Mit anderen Worten umspannt das Implantat im Expansionszustand E ein größeres Raumvolumen als im Ausgangszustand A.

In Fig. 1 sind die Enden 8a,b jedes Stegs 6 an den Endstücken 4a,b um einen Ausgangswinkel β von 180° in Umfangsrichtung versetzt zueinander angeordnet. Die maximale Expansion zwischen Ausgangszustand A und Expansionszustand E gemäß Fig. 2 ist dann bei einer Relativdrehung der Endstücke 4a,b um 180° in Richtung der Pfeile 16a,b gegenüber der Situation in Fig. 1 erreicht. In Fig. 2 liegen daher die Enden 8a,b jedes Steges 8 auf jeweils derselben Umfangsposition, d.h. deren Winkelversatz ist dann 0°.

Die Stege 6 in der Seitenansicht des Implantats 2 gemäß Fig. 2 erstrecken sich also geradlinig, d.h. parallel zur Mittellängsachse 10 von ihrem einen zu ihrem anderen Ende 8a,b. Die Stege 6 weisen an ihren Enden 8a,b Schwächungsstellen 20 auf, die wie Gelenke wirken. Die Schwächungsstellen 20 dienen dazu, dass bei einer gegenseitigen Verdrehung der Endstücke 4a,b sich die Enden 8a,b der Stege gegenüber den Endstücke 4a,b verdrehen können. Die Schwächungsstellen 20 sind so ausgestaltet, dass bei einer Verdrehung der Endstücke 4a,b die sich zwischen zwei Schwächungsstellen 20 eines Steges 6 erstreckenden Stegbereiche nicht oder zumindest nur geringfügig verwinden, so dass die Stege 6 im wesentlichen Ihre ursprüngliche Form behalten. Die Verformung der Schwächungsstellen 20 erfolgt außerdem gleichmäßig, so dass sich die Ausrichtung der Außenseiten 14 in einem axialen Mittelbereich 22 des Implantats 2 zwischen Ausgangszustand A und Expansionszustand E nahezu nicht ändert. Ein Normalenvektor in Form der Flächennormalen 24 der Außenseite 14 im Mittelbereich 22 besitzt daher lediglich eine Radial- und eine Axial-, aber keine Komponenten in Umfangsrichtung.

Die Außenseiten des Implantats 2 bilden im Expansionszustand E etwa einen Teil der Hüllfläche eines Tonnenkörpers. Dadurch ist gewährleistet, dass die Stege 6 bei der gegenseitigen Verdrehung der Endstücke 4a,b mit ihrer Außenseite zumindest im Mittelbereich 22 großflächig das sie umgebene Spongiosa-Gewebe beaufschlagen und zur Seite drängen können. Außerdem ist verhindert, dass die Stege 6 mit kantigen Rändern in Form von Kanten 26 Spongiosagewebe abschaben. Dies könnte nämlich dann der Fall sein, wenn sich die Stege 6 in sich verwinden und so ihre Kanten 26 eine Schabwirkung gegenüber dem Spongiosagewebe ausüben würden.

Um die beschriebene Ausrichtung der Stege 6 während der Expansion des Implantats 2 zu gewährleisten, sind die Schwächungsstellen 20 aus zwei unterschiedlich gestalteten Teilen gebildet. Der erste Teil wird durch eine verringerte Wanddicke des Steges in Radialrichtung und der zweite Teil durch eine verringerte Breite der Stege 6 in Umfangsrichtung gebildet.

Die beiden Endstücke 4a,b sind so ausgestaltet, dass sie mit dem Werkzeug 18 formschlüssig in Eingriff gebracht und mit Hilfe dessen gegeneinander verdreht werden können. Hierzu weist das Endstück 4a eine zentrale, von der Kreisform abweichende, im Beispiel etwa sternförmige Ausnehmung auf, die ein erstes, in Umfangsrichtung wirksames Eingriffsmittel 28a darstellt.

Fig. 4 zeigt eine Draufsicht von vorne auf das Implantat 2, in der das Eingriffsmittel 28a und das formschlüssig darin eingreifende Werkzeug 18 zu sehen sind.

Das hintere Endstück 4b ist so gestaltet, dass das Handhabungswerkzeug 18 an dessen Außenumfang bzw. der äußeren Stirnseite mit einem in Drehrichtung wirksamen Formschluss angreifen kann. Dazu weist das Endstück 4b ein Eingriffsmittel 28b auf. Dieses wird gebildet durch in Axialrichtung verlaufende schlitzförmige Ausnehmungen als Formschlusselement 30. Alternativ könnte auch - nicht dargestellt - das Endstück 4b entsprechend dem Endstück 4a eine entsprechende Ausnehmung aufweisen.

Im Beispiel ist jedoch dort eine zusätzliche kreisrunde, keinen Formschluss erlaubende Durchtrittsöffnung 32 vorgesehen, durch die ein Teil des Werkzeugs 18 zum Eingriffsmittel 28a gelangen kann.

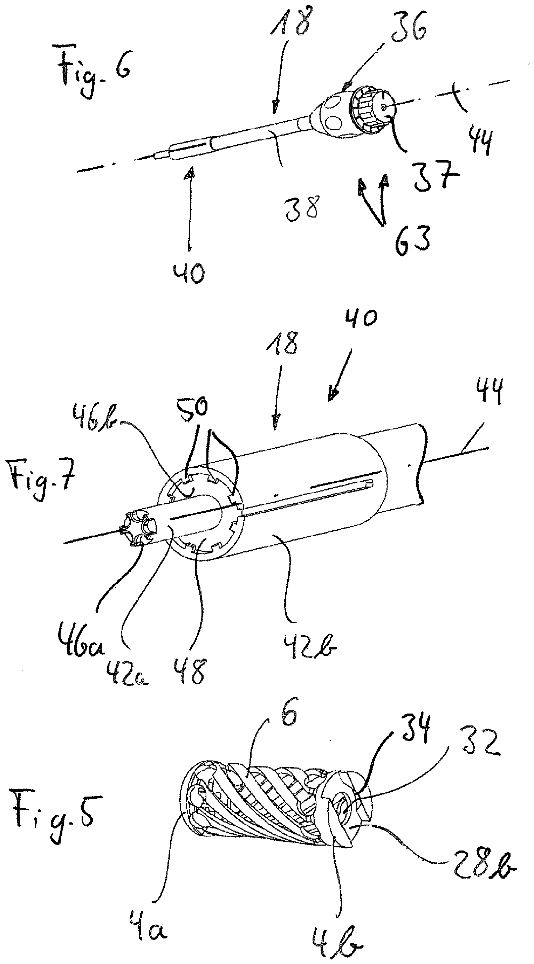

Fig. 5 zeigt ein alternatives Eingriffsmittel 28b am Endstück 4b in Form einer Quernut. Dieses ist außerdem so gestaltet, dass sich damit auch ein in Axialrichtung wirksamer Formschluss mit dem Werkzeug 18 herstellen lässt. Hierzu ist ein Hinterschnitt 34 vorgesehen.

Gemäß Fig. 6 weist das Werkzeug 18 weist einen Handgriff 36 und einen daran angebrachten länglichen Grundkörper 38 auf. Dem Handgriff 36 gegenüberliegend weist dieser ein Betätigungsende 40 auf. Dort ist, siehe Fig. 3, das Implantat 2 aufsteckbar. Somit bildet das Betätigungsende auch eine Aufnahme 41 für das Implantat.

Das Betätigungsende 40 weist gemäß Fig. 7 einen ersten Abschnitt 42a und einen zweiten Abschnitt 42b auf, die koaxial bezüglich einer Mittellängsachse 44 zueinander angeordnet und gegeneinander verdrehbar sind.

Der erste Abschnitt 42a hat einen kleineren Durchmesser als der zweite Abschnitt 42b und trägt an seiner Stirnseite ein zum Eingriffsmittel 28a komplementäres Formstück, das ein erstes Drehmittel 46a bildet. Dessen Zugang zum Eingriffsmittel 28a erfolgt, indem der Abschnitt 42a durch die Durchtrittsöffnung 32 geführt wird. Die dem ersten Abschnitt 42a zugewandte Stirnseite 48 des zweiten Abschnitts 42b weist eine Formgebung in Form eines Drehmittels 46b auf, die einen drehwirksamen Formschluss mit dem Eingriffsmittel 28b erlaubt. Hierzu ist eine das Endstück 4b aufnehmende axiale Ausnehmung mit radial nach innen stehenden Vorsprüngen 50 vorgesehen, die in die schlitzförmigen Ausnehmungen 30 eingreifen. Bei dem Werkzeug ist z.B. das eine Drehmittel 46a mit einem stirnseitigen Rändelrad 37, das andere Drehmittel drehfest mit dem Handgriff verbunden. Durch jeweilige Rotation von Handgriff 36 und Rändelrad 37 können dann die Drehmittel 46a,b individuell betätigt werden. Handgriff 36 und Rändelrad 37 stellen damit Betätigungselemente 63 für die Drehmittel 46a,b dar.

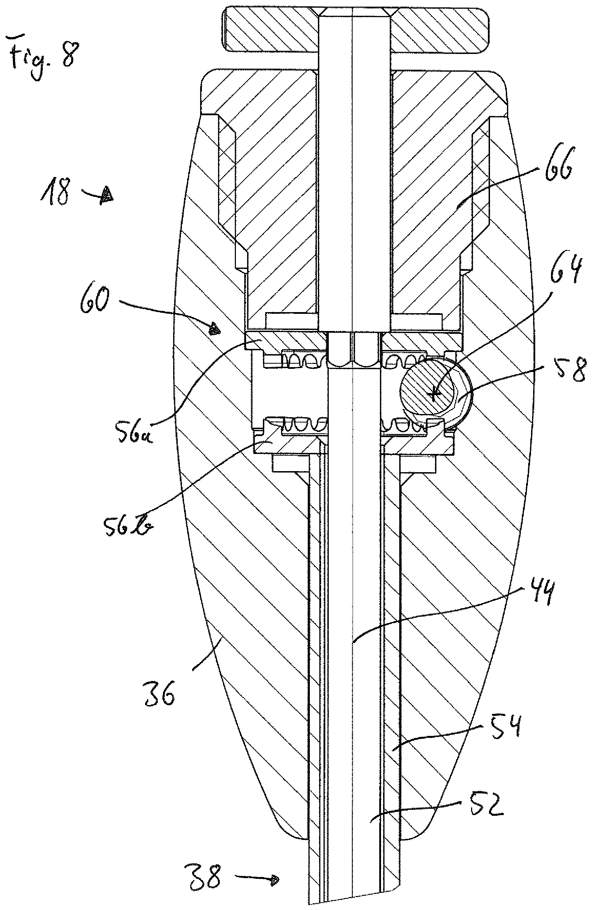

Bei einer bevorzugten Variante gemäß Fig. 8 ist das Werkzeug 18 so ausgebildet, dass sich die Abschnitte 42a,b gegensinnig bezüglich des Handgriffes 36 drehen lassen, wobei die Verdrehung stets synchron und zwangsgeführt um den gleichen Winkelversatz erfolgt.

Hierzu ist der Abschnitt 42a drehfest mit einer Innenspindel 52, und der Abschnitt 42b mit einem diese koaxial umgebenden Rohr 54 verbunden, die zusammen den Grundkörper 38 bilden. Im Inneren des Handgriffs 36 ist, wiederum drehfest, die Innenspindel 52 auf ein Zahnrad 56a, und das Rohr 54 auf ein Zahnrad 56b geführt. Diese sind zusammen mit einer Gewindeschnecke 58 zu einem gegenläufigen Schneckengetriebe 60 gekoppelt. Die Innenspindel 52 und Rohr 54 sind hierbei im Handgriff 36 um die Mittellängsachse 44 drehbar, die Gewindeschnecke 58 bezüglich dieser drehfest angeordnet. Die Gewindeschnecke ist jedoch um eine zur Mittellängsachse 44 senkrechte Drehachse 64 drehbar gelagert. Das Schneckengetriebe 60 ist im Handgriff 36 durch eine Fixiermutter 66 gehalten. Durch deren Entfernung kann das gesamte Werkzeug 18 zerlegt, gereinigt und sterilisiert werden.

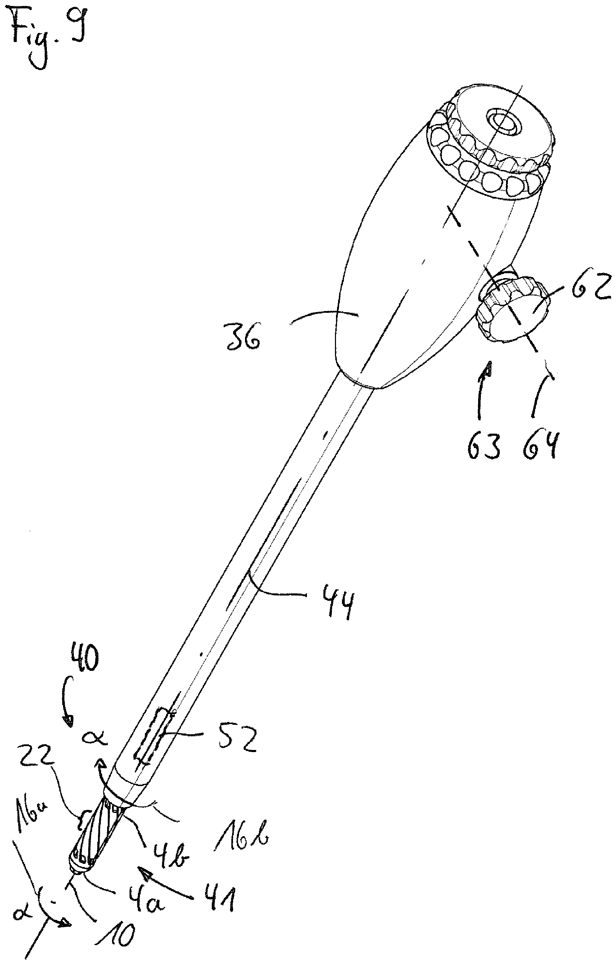

Fig. 9 zeigt die Außenansicht des Werkzeugs 18 mit einem mit der Gewindeschnecke verbundenen Drehknopf 62. Ein alternatives Implantat 2 (s.u.) ist aufgesetzt und befindet sich mit seinen Eingriffsmittelim 28a,b in Formschluss mit den Drehmitteln 46a,b. Wird nun der Handgriff 36 festgehalten, und der Drehknopf 62 um die Drehachse 64 gedreht, so bewegen sich die Innenspindel 52 und mit dieser das Endstück 4a in Richtung des Pfeils 16a um die zusammenfallenden Mittellängsachsen 10 und 44 um einen bestimmten Drehwinkel α. Gleichzeitig und gegensinnig um den gleichen Drehwinkel α drehen sich das Rohr 54 und das Endstück 4b in Richtung des Pfeils 16b. Diese streng gegenläufige Verwindung hat zur Folge, dass sich der Mittelbereich 22 in Relation zum Handgriff 36 nahezu nicht in Umfangsrichtung bewegt, sondern lediglich radial expandiert. Wird also der Handgriff 36 bezüglich eines nicht dargestellten Patienten nicht gedreht, erfährt auch der Mittelbereich 22 keine Drehung im Patienten, sondern nur eine radiale Expansion ohne Schabwirkung. Im diesen Ausführungsbeispiel ist lediglich der Drehknopf 62, nicht jedoch der Handgriff 36 ein Betätigungselement 63 für die Drehmittel 46a,b.

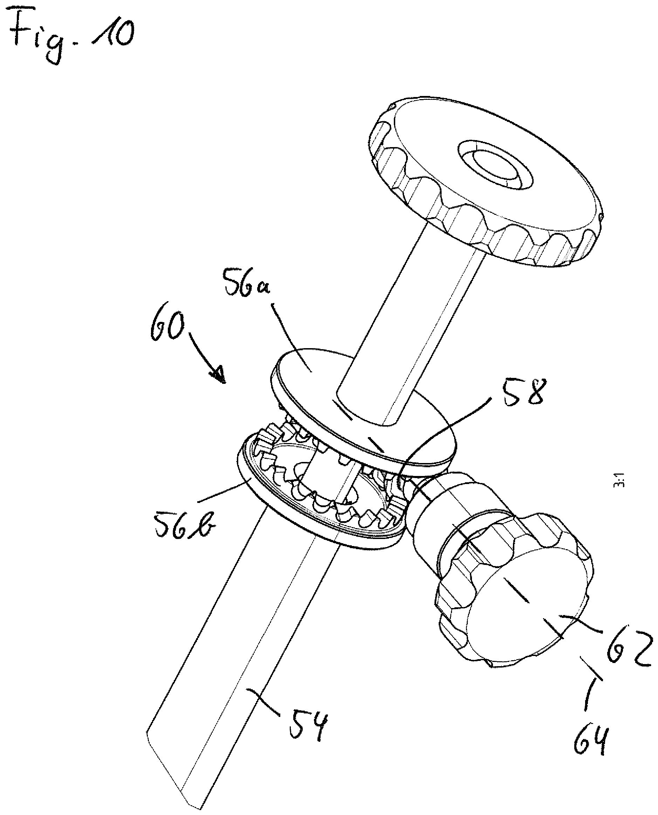

Fig. 10 zeigt nochmals die kinematischen Zusammenhänge zwischen Drehknopf 62 und dem Schneckengetriebe 60 unter Weglassung des Handgriffs 36.

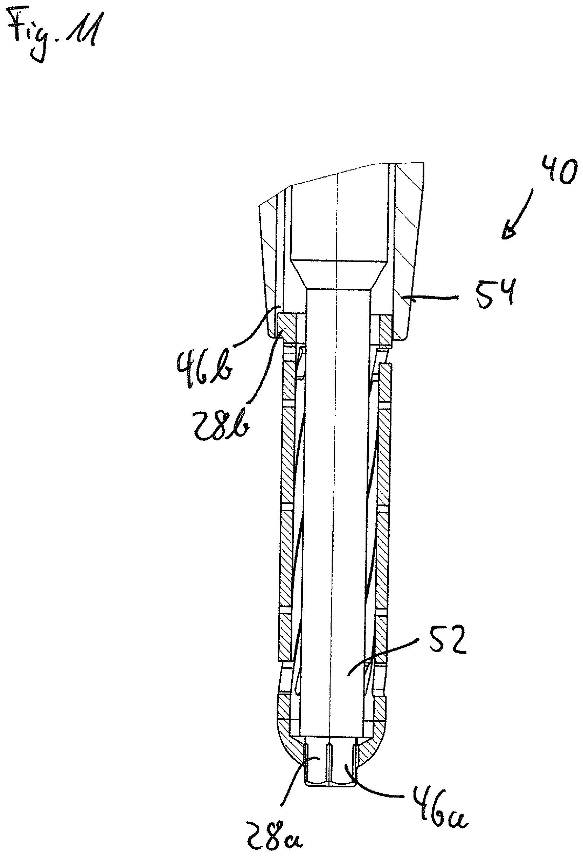

Fig. 11 zeigt im Detail das Betätigungsende 40 des Werkzeuges 18, insbesondere den Eingriff des Drehmittels 46a an der Innenspindel 52 in das Eingriffsmittel 28a, und den Eingriff des Drehmittels 46b am Rohr 54 in das Eingriffsmittel 28b.

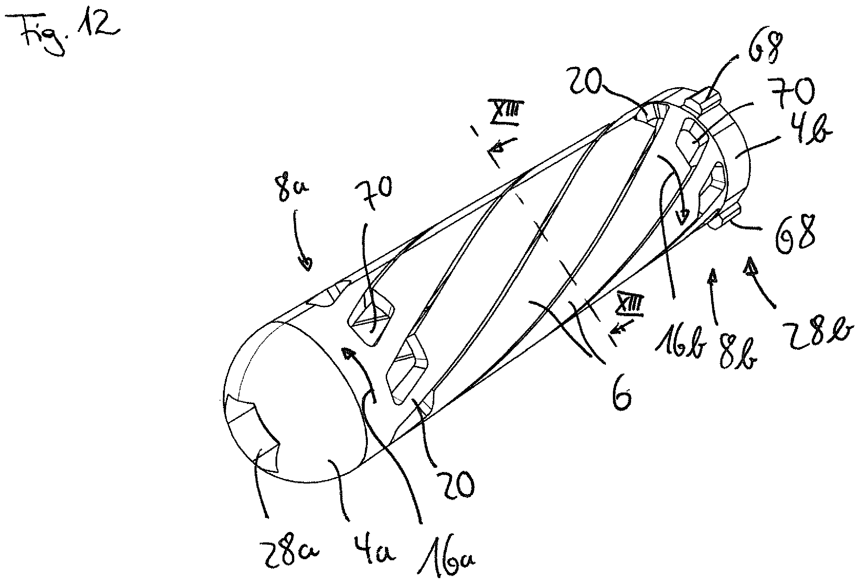

Die Figs. 9 und 11 zeigen bereits ein alternatives Implantat 2 gemäß Fig. 12. Dieses unterscheidet sich von der vorherigen Ausführungsform insbesondere durch die Gestaltung des Eingriffsmittels 28b in Form von über den Umfang verteilten, radial nach außen abstehenden Nasen als Formschlusselement 30, sowie des in sechseckiger Form ausgeführten Eingriffsmittel 28a. Außerdem weisen die Stege 6 an ihren Enden 8a,b alternativ gestaltete Schwächungsstellen 20 auf. Im Gegensatz zum ersten Ausführungsbeispiel ist die radiale Wandstärke der Stege 6 nicht geschwächt, d.h. auch an den Schwächungsstellen 20 vollständig durchgängig erhalten. Anstelle von zwei - in Umfangsrichtung gesehen - seitlichen Ausnehmungen ist außerdem nur an einer Seite der Stege 6 eine entsprechende Ausnehmung gestaltet, um die Schwächungsstelle 20 zu gestalten. Die der Schwächungsstelle 20 gegenüberliegende Seite der Stege 6, sowie die radiale Innen- und Außenfläche der Stege ist somit bündig durchgehend ausgestaltet. Die entsprechenden Ausnehmungen 70 sind außerdem jeweils in diejenigen Seite der Stege 6 eingebracht, welche in die Drehrichtung des betreffenden Endstückes 4a,b, also in Richtung der Pfeile 16a und 16b weisen. Der Windungssinn der Stege 6 ist auch gegenüber dem ersten Ausführungsbeispiel entgegengesetzt, weshalb auch die Pfeile 16a,b in entgegengesetzte Richtungen weisen.

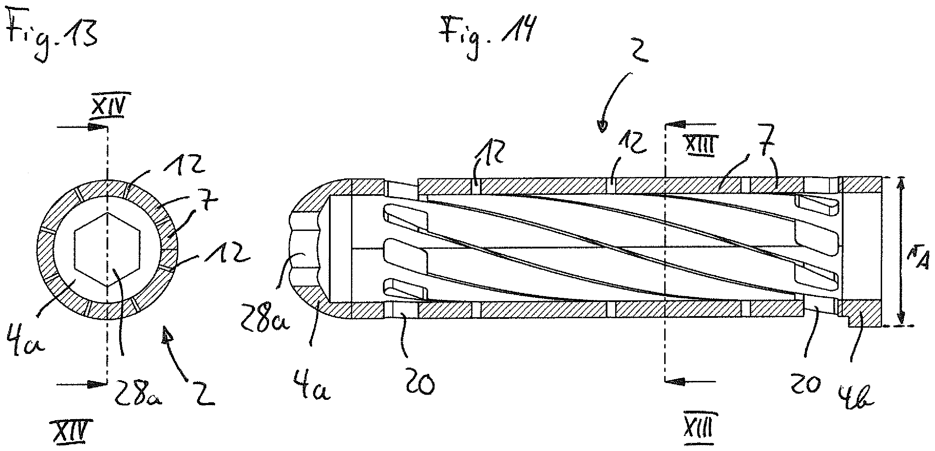

Fig. 13 zeigt eine Schnittdarstellung des Implantates 2 aus Fig. 12 in Richtung des Pfeils 13, Fig. 14 einen Längsschnitt durch das entsprechende Implantat 2, welches gegenüber der ersten Ausführungsform insbesondere die fehlende Schwächung bezüglich der Wandstärke der Stege 7 und der Endstücke 4a,b an den Schwächungsstellen 20 zeigt.

Fig. 14 ist außerdem zu entnehmen, wie das Implantat 2 z.B. einstückig herstellbar ist, indem dieses aus einem entsprechenden stabförmigen Vollmaterial zunächst vom Endstück 4b her zentral hohlgebohrt wird, das Endstück 4a vorne halbrund abgedreht wird und sodann das Eingriffsmittel 28a sowie die Restspalte 12 und die Schwächungsstellen 20 z.B. durch Laserschneiden aus dem Vollmaterial abgetragen werden.

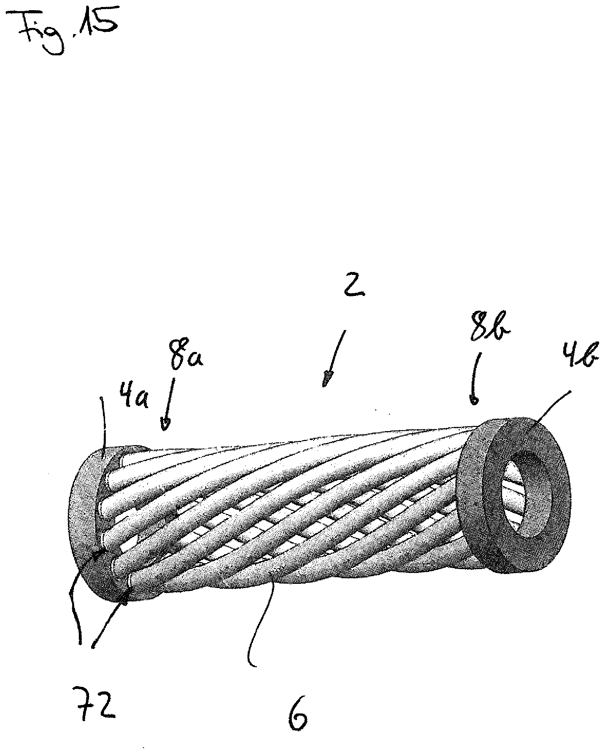

Fig. 15 zeigt ein weiteres alternatives Implantat 2 im Prinzipbild. Die Eingriffsmittel 28a,b sind der Einfachheit halber weggelassen. Eine gelenkige Verbindung zwischen den Stegen 6 und den Endstücke 4a,b kann hier auch erreicht werden, wenn anstelle von wie Gelenke wirkenden Schwächungsstellen 20 tatsächliche Gelenke 72 im Bereich der Enden 8a,b der Stege 6 - auch Fixierpunkte genannt - vorgesehen sind. Die Gelenke können etwa als Kugelgelenke ausgeführt sein, bei denen das Ende 8a,b eines Stegs 6 eine Gelenkkugel trägt, die in einer Gelenkpfanne in den Endstücken 4a,b gehalten sind. Auch müssen die Stege nicht zwangsläufig, wie oben beschrieben, eckig im Querschnitt ausgestaltet sein. Die Stege 6 können, wie hier gezeigt, auch als Stangen mit z.B. kreisrunden Querschnitt ausgebildet sein

## Patentansprüche

1. Implantat (2) für die Kyphoplastie,
- mit zwei bezüglich einer Mittellängsachse (10) koaxial, und mit einem Axialabstand (1) zueinander angeordneten Endstücken (4a,b),
- mit einem die Endstücke (4a,b) verbindenden, und die Mittellängsachse (10) konzentrisch umgebenden Käfig (7), wobei dieser
- eine Mehrzahl von beide Endstücke (4a,b) verbindenden Stegen (6) enthält,
**dadurch gekennzeichnet, dass**
die beiden Enden (Ba,b) eines jeden Steges (6) in einem Ausgangszustand (A) des Implantats (2) in Umfangsrichtung bezüglich der Mittellängsachse (10) um einen gleichen Ausgangswinkel (ß) gegeneinander versetzt sind,
- wobei das Implantat (2) durch eine gegensinnige Rotation der Endstücke (4a,b) um die Mittellängsachse (10), bei der sich die Umfangspositionen der Enden (8a,b) jedes Steges (6) aufeinander zu bewegen, in einen Expansionszustand (E) bringbar ist.

2. Implantat (2) nach Anspruch 1, bei dem im Ausgangszustand (A) die Enden (8a,b) jedes Steges (6) in Umfangsrichtung bezüglich der Mittellängsachse (10) um einen Ausgangswinkel (ß) von 180° versetzt sind.

3. Implantat (2) nach einem der vorhergehenden Ansprüche, bei dem die Enden (8a,b) eines Steges (6) in einer eine Verformung begünstigenden Weise gegenüber dem restlichen Steg (6) geschwächt sind.

4. Implantat (2) nach Anspruch 3, bei dem der Querschnitt der Enden (8a,b) der Stege (6) gegenüber dem Querschnitt des restlichen Steges (6) verjüngt ist.

5. Implantat (2) nach Anspruch 4, bei dem der Querschnitt der Enden (8a,b) der Stege (6) durch eine an einer Seite des Steges (6) vorhandene Ausnehmung (70) verjüngt ist.

6. Implantat (2) nach einem der vorhergehenden Ansprüche, bei dem in einem axialen Mittelbereich (22) die Flächennormale (24) der radial nach außen gerichteten Fläche jedes Steges (6) lediglich eine Radial- und Axialkomponente aufweist.

7. Implantat (2) nach einem der vorhergehenden Ansprüche, bei dem ein erstes Endstück (4a) ein in Umfangsrichtung wirksames erstes Eingriffsmittel (28a), und ein zweites Endstück (4b) eine Durchtrittsöffnung (32) und ein in Umfangsrichtung wirksames zweites Eingriffsmittel (28b) für ein Werkzeug (18) aufweist.

8. Implantat (2) nach Anspruch 7, bei dem das erste Eingriffsmittel (28a) eine Ausnehmung im ersten Endstück (4a) und das zweite Eingriffsmittel (28b) ein Formschlusselement (30) am Außenumfang des zweiten Endstücks (4b) ist.

9. Implantat (2) nach einem der vorhergehenden Ansprüche, das einstückig ausgeführt ist.

10. Werkzeug (18) für die Kyphoplastie, für ein Implantat (2) nach einem der Ansprüche 7 bis 8,
- mit einem Handgriff (36),
- und einem stabförmigen, eine Mittellängsachse (44) aufweisenden Grundkörper (38), an dessen einem Ende der Handgriff (36) und an dessen anderem Ende eine Aufnahme (41) für das Implantat (2) angeordnet ist,
- mit zwei, an je einem Eingriffsmittel (28a,b) formschlüssig angreifenden Drehmitteln (46a,b), die bezüglich der Mittellängsachse (44) des Grundkörpers (38) gegeneinander rotierbar sind, wobei die Drehmittel (46a,b) bezüglich des Handgriffs (36) gegensinnig rotierbar, und bezüglich eines jeweils gleichen Winkelversatzes zwangsgeführt sind.

11. Werkzeug (18) nach Anspruch 10, mit einem mindestens ein Drehmittel (46a,b) antreibenden Betätigungselement (63).

12. Werkzeug (18) nach Anspruch 11, bei dem das Betatigungselement (63) ein um eine quer zur Mittellängsachse (44) verlaufende Drehachse (64) drehbarer Drehknopf (62) ist.

## Claims

1. Implant (2) for kyphoplasty,
- having two end pieces (4a, b) arranged coaxially with respect to a central longitudinal axis (10) and having an axial distance (1) relative to each other,
- having a cage (7) that connects the end pieces (4a, b) and concentrically surrounds the central longitudinal axis (10), wherein this cage (7)
- contains a plurality of webs (6) connecting the two end pieces (4a, b),
**characterised in that**,
in an initial state (A) of the implant (2), the two ends (8a, b) of each web (6) are offset from each other by the same initial angle (ß) in the peripheral direction with respect to the central longitudinal axis (10),
- wherein the implant (2) is able to be brought into an expansion state (E) by an opposing rotation of the end pieces (4a, b) around the central longitudinal axis (10) in which the peripheral positions of the ends (8a, b) of each web (6) are moved towards one another.

2. Implant (2) according to claim 1, wherein, in the initial state (A), the ends (8a, b) of each web (6) are offset by an initial angle (ß) of 180° in the peripheral direction with respect to the central longitudinal axis (10).

3. Implant (2) according to one of the preceding claims, wherein the ends (8a, b) of a web (6) are weakened in comparison to the rest of the web (6) in a manner that favours a deformation.

4. Implant (2) according to claim 3, wherein the cross-section of the ends (8a, b) of the webs (6) is tapered in comparison to the cross-section of the rest of the web (6).

5. Implant (2) according to claim 4, wherein the cross-section of the ends (8a, b) of the webs (6) is tapered by a recess (70) present on one side of the web (6).

6. Implant (2) according to one of the preceding claims, wherein, in an axial central region (22), the surface normal (24) of the radially outwardly aligned surface of each web (6) only has one radial and axial component.

7. Implant (2) according to one of the preceding claims, wherein a first end piece (4a) has a first engaging means (28a) that acts in the peripheral direction, and a second end piece (4b) has a through-opening (32) and a second engaging means (28b) that acts in the peripheral direction for a tool (18).

8. Implant (2) according to claim 7, wherein the first engaging means (28a) is a recess in the first end piece (4a) and the second engaging means (28b) is a positive locking element (30) on the outer periphery of the second end piece (4b).

9. Implant (2) according to one of the preceding claims that is implemented to be one piece.

10. Tool (18) for kyphoplasty, for an implant (2) according to one of claims 7 to 8,
- having a handle (36),
- and a rod-shaped main body (38) that has a central longitudinal axis (44), the handle (36) being arranged on one end of said main body (38) and a receiver (41) for the implant (2) on the other end of said main body (38),
- having two rotation means (46a, b) which each engage with an engaging means (28a, b) in a positive-locking manner and are able to be rotated against each other with respect to the central longitudinal axis (44) of the main body (38), wherein the rotation means (46a, b) are able to be rotated in opposite directions to each other with respect to the handle (36) and are positively guided with respect to a respective equal angle offset.

11. Tool (18) according to claim 10, having an actuation element (63) that drives at least one rotation means (46a, b).

12. Tool (18) according to claim 11, wherein the actuation element (63) is a rotating button (62) that is able to rotate around a rotation axis (64) that runs perpendicular to the central longitudinal axis (44).

## Revendications

1. Implant (2) pour la cyphoplastie,
- comprenant deux embouts (4a, b) montés coaxialement par rapport à un axe (10) longitudinal médian et à une distance (1) axiale l'un de l'autre,
- comprenant une cage (7) reliant les embouts (4a, b) et entourant concentriquement l'axe (10) longitudinal médian, dans lequel celui-ci
- comporte une pluralité d'entretoises (6) reliant les deux embouts (4a, b),
**caractérisé en ce que**
les deux extrémités (Ba, b) d'une entretoise (6) sont, dans un état (A) initial de l'implant (2), décalées, l'une par rapport à l'autre, d'un même angle (β) initial dans la direction périphérique par rapport à l'axe (10) longitudinal médian,
- dans lequel l'implant (2) peut, par une rotation en sens contraire des embouts (4a, b) autour de l'axe (10) longitudinal médian, dans laquelle les positions périphériques des extrémités (8a, b) de chaque entretoise (6) se déplacent l'une par rapport à l'autre, être mis dans un état (E) d'expansion.

2. Implant (2) suivant la revendication 1, dans lequel, dans l'état (A) initial, les extrémités (8a, b) de chaque entretoise (6) sont décalées d'un angle (β) initial de 180° dans la direction périphérique par rapport à l'axe (10) longitudinal médian.

3. Implant (2) suivant l'une des revendications précédentes, dans lequel les extrémités (8a, b) d'une entretoise (6) sont affaiblies par rapport au reste de l'entretoise (6), d'une façon favorisant une déformation.

4. Implant (2) suivant la revendication 3, dans lequel la section transversale des extrémités (8a, b) des entretoises (6) est rétrécie par rapport à la section transversale du reste de l'entretoise (6).

5. Implant (2) suivant la revendication 4, dans lequel la section transversale des extrémités (8a, b) des entretoises (6) est rétrécie par un évidement (70) présent sur un côté de l'entretoise (6).

6. Implant (2) suivant l'une des revendications précédentes, dans lequel, dans une partie (22) médiane axiale, les normales (24)) à la surface, dirigées radialement vers l'extérieur de chaque entretoise (6), ont simplement une composante radiale et axiale.

7. Implant (2) suivant l'une des revendications précédentes, dans lequel un premier embout (4a) a un premier moyen (28a) de mise en prise efficace dans la direction périphérique et un deuxième embout (4b) a une ouverture (32) de passage et un deuxième moyen (28b) de mise en prise efficace dans la direction périphérique pour un outil (18).

8. Implant (2) suivant la revendication 7, dans lequel le premier moyen (28a) de mise en prise a un évidemment du premier embout (4a) et le deuxième moyen (28b) de mise en prise a un élément (30) à complémentarité de forme sur le pourtour extérieur du deuxième embout (4b).

9. Implant (2) suivant l'une des revendications précédentes, qui est d'une seule pièce.

10. Outil (18) pour la cyphoplastie, pour un implant (2) suivant l'une des revendications 7 à 8,
- comprenant une poignée (36),
- et un corps (38) de base en forme de barreau et ayant un axe (44) longitudinal médian, corps à l'une des extrémités duquel la poignée (36) est montée et à l'autre extrémité duquel est prévu un logement (41) pour l'implant (2),
- comprenant deux moyens (46a, b) attaquant, en complémentarité de forme, respectivement un moyen (28a, b) de mise en prise et pouvant tourner, l'un par rapport à l'autre, par rapport à l'axe (44) longitudinal médian du corps (38) de base, les moyens (46a, b) de rotation pouvant tourner en sens contraire par rapport à la poignée (36) et étant guidés de manière forcée en ce qui concerne un même décalage angulaire.

11. Outil (18) suivant la revendication 10, comprenant au moins un élément (63) d'actionnement entraînant un moyen (46a, b) de rotation.

12. Outil (18) suivant la revendication 11, dans lequel l'élément (63) d'actionnement est un bouton (62) tournant, pouvant tourner autour d'un axe (64) de rotation, s'étendant transversalement à l'axe (44) longitudinal médian.
